# FASCICULE DE BREVET EUROPEEN

(11) **EP 2 332 556 B1**
(45) Date de publication et mention de la délivrance du brevet: **16.09.2015**
(21) Numéro de dépôt: 10194022.9
(22) Date de dépôt: 07.12.2010
(51) Int. Cl.: A61K 36/06, A61K 45/06, A61K 31/192, A61P 1/04, C12N 1/16

(54) **Levures pour le traitement des ulcères gastriques ou intestinaux induits par des agents gastrotoxiques**
Verwendung von Hefe für die Behandlung von Magengeschwüren oder intestinalen Geschwüren induziert durch gastrotoxische Substanzen
Use of yeasts for treating stomach or intestinal ulcers induced by gastrotoxic agents

(30) Priorité: 08.12.2009 FR 0958734
(43) Date de publication de la demande: 15.06.2011
(73) Titulaire: Biocodex, 94250 Gentilly (FR)
(72) Inventeur: Girard, Philippe, 60280, MARGNY-LES-COMPIEGNE (FR); Le Guern, Marie-Emmanuelle, 60200, COMPIEGNE (FR); Verleye, Marc, 60190, REMY (FR); Hublot, Bernard, 60200, COMPIEGNE (FR)
(74) Mandataire: Vial, Lionel

(56) Documents cités:
- WO-A1-2004/037232
- WO-A1-2007/150052
- US-A1- 2006 140 973
- US-B1- 6 649 383
- GOTTELAND M. ET AL.: "Systematic reviwe : are probiotics useful in controlling gastric colonization by Helicobacter pylori?", ALIMENT. PHARMACOL. THER., vol. 23, 2006, pages 1077-1086, XP002591187,

## Description

### Domaine de l'invention

La présente invention concerne un médicament pour la prévention ou le traitement des ulcères gastriques ou intestinaux.

### Arrière-plan technique

Les ulcères gastriques ou intestinaux se manifestent essentiellement par une perte de substance d'un revêtement épithélial muqueux sans tendance à la cicatrisation spontanée et par une lésion de la paroi gastrique ou intestinale, notamment duodénale. On considère généralement que l'ulcère gastrique ou intestinal, notamment duodénal, résulte d'un déséquilibre entre des facteurs d'agression, tels que les sécrétions gastriques acide et peptique, et des facteurs de défense, tels que la synthèse des prostaglandines, le mucus et l'épithélium de surface.

Outre *Helicobacter pylori,* la principale source d'ulcères gastro-intestinaux est médicamenteuse. Ainsi, l'aspirine et les anti-inflammatoires non-stéroïdiens (AINS), tel que l'ibuprofène, inhibent notamment les cyclo-oxygénases, entraînant une diminution de la synthèse des prostaglandines et la sécrétion de mucus gastro-intestinal protecteur. En conséquence, la muqueuse gastro-intestinale se trouve exposée aux attaques acides des sécrétions gastriques.

Des AINS présentant une sélectivité accrue vis-à-vis de la cyclo-oxygénase de type 2 (COX-2), principalement inflammatoire, par rapport à la COX-1, formant la classe médicamenteuse des COXIB, ont été développés. Toutefois, les études cliniques réalisées jusqu'à présent indiquent que les COXIB présentent également un risque accru d'apparition d'ulcères gastro-intestinaux (Yeomans (2002) Journal of Gastroenterology and Hepatology 17:488-494), ce qui pourrait être un reflet du rôle de la COX-2 dans la réparation des lésions muqueuses (Halter et al. (2001) Gut 49:443-453).

Ainsi, le problème des ulcères gastro-intestinaux liés aux AINS se pose de manière particulièrement aiguë pour les individus traités sur le long terme par ces composés, par exemple dans le cadre de la prise en charge de douleurs et d'inflammations articulaires.

A l'heure actuelle, les principaux traitements utilisés pour les ulcères gastro-intestinaux liés aux AINS font appel à deux types de composés (Yeomans (2002) Journal of Gastroenterology and Hepatology 17:488-494 ; Florent et al. (1990) Acta Endoscopica 20: 427-439) :
(i) des inhibiteurs de sécrétions gastriques : inhibiteurs de la pompe à proton, tel que l'oméprazole, ou antagonistes des récepteurs H2 à l'histamine, tels que la cimétidine et la ranitidine ;
(ii) des stimulateurs de la sécrétion de mucus, comme les prostaglandines, et notamment le misoprostol (Graham et al. (1988) Lancet 2:1277-1280).

Toutefois, aucun de ces traitements n'est pleinement satisfaisant.

*Saccharomyces boulardii* (Ultra-Levure®) est une souche particulière de la levure *Saccharomyces cerevisiae.* Ce probiotique est principalement indiqué en complément de la réhydratation pour le traitement des diarrhées. Son utilité a été notamment établie chez l'enfant (Villarruel et al. (2007) Acta Paediatr 96:538-541 ; Szajewska et al. (2007) Aliment Pharmacol Ther 25:257-264) et pour les diarrhées liées à la prise d'antibiotiques (Surawicz et al. (1989) Gastroenterology 96:981-988; Kotowska et al. (2005) Aliment Pharmacol Ther 21:583-590) ou aux infections à *Clostridium difficile* (Surawicz et al. (2000) Clin Infect Dis 31:1012-1017).

### Résumé de l'invention

La présente invention découle de la mise en évidence inattendue, par les inventeurs, que *Saccharomyces boulardii* permettait de diminuer le nombre et la surface d'ulcères gastriques liés à l'ibuprofène chez le rat.

Ainsi, la présente invention concerne des cellules de levure pour leur utilisation dans la prévention ou le traitement des ulcères gastriques ou intestinaux induits par un principe actif de médicament ayant une activité gastrotoxique et susceptible de provoquer des ulcères gastriques ou intestinaux comme effet secondaire.

La présente invention concerne également une composition pharmaceutique comprenant à titre de substances actives :
- des cellules de levure de l'espèce *Saccharomyces boulardii,* éventuellement lyophylisées, et
- au moins un principe actif de médicament ayant une activité gastrotoxique et susceptible de provoquer des ulcères gastriques ou intestinaux comme effet secondaire,
éventuellement en association avec un véhicule pharmaceutiquement acceptable.

La présente invention concerne également, une trousse comprenant :
- dans un premier compartiment des cellules de levure ;
- dans un deuxième compartiment au moins un principe actif de médicament ayant une activité gastrotoxique et susceptible de provoquer des ulcères gastriques ou intestinaux comme effet secondaire.

Dans un mode de réalisation particulier, la trousse telle que définie ci-dessus est utilisée à titre de médicament ou de produit à visée thérapeutique.

Par ailleurs, dans un mode de réalisation particulier des cellules de levure, de l'utilisation, de la méthode, de la composition pharmaceutique ou à visée thérapeutiques, ou de la trousse, telles que définies ci-dessus, aucun composé additionnel destiné au traitement des ulcères gastriques ou intestinaux, tel que l'oméprazole par exemple, n'est associé aux cellules de levure selon l'invention.

### Description des figures

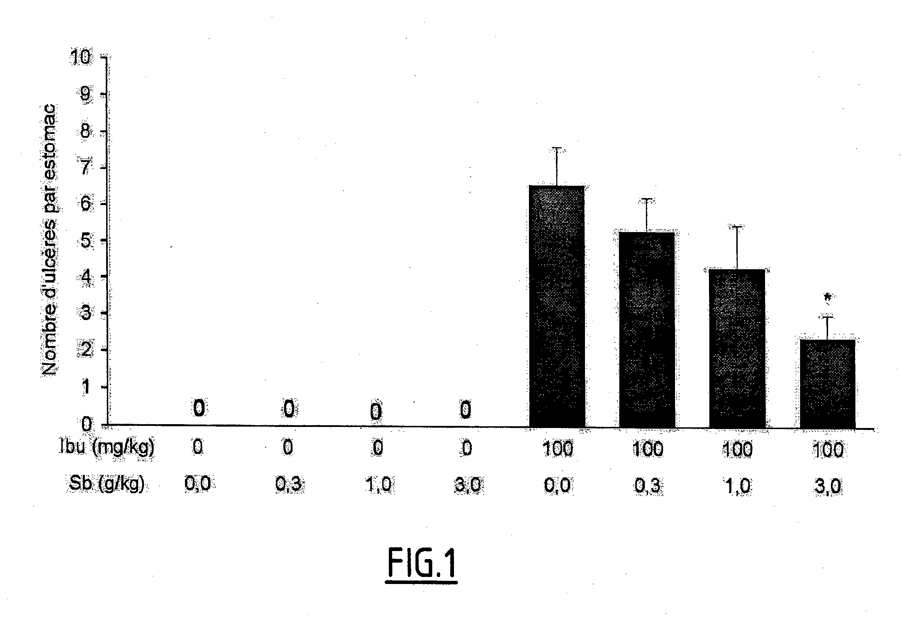

La figure 1 représente l'effet de *Saccharomyces boulardii* (Sb, voie orale) sur le nombre d'ulcères (axe des ordonnées) induits par une administration répétée d'ibuprofène (Ibu, voie orale) pendant 6 jours consécutifs. Sb est donné pendant 6 jours, 1 heure avant l'ibuprofène et on note le nombre d'ulcères le sixième jour.

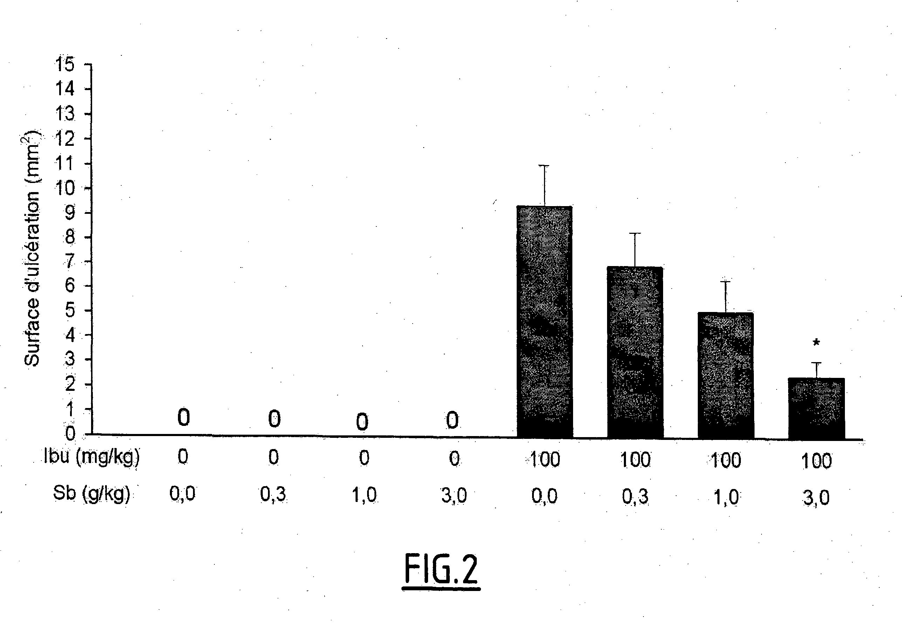

La figure 2 représente l'effet de *Saccharomyces boulardii* (Sb, voie orale) sur la surface d'ulcération (axe des ordonnées, mm²) induite par une administration répétée d'ibuprofène (Ibu, voie orale) pendant 6 jours consécutifs. Sb est donné pendant 6 jours, 1 heure avant l'ibuprofène et on mesure la surface d'ulcération le sixième jour.

### Description détaillée de l'invention

Les ulcères gastriques ou intestinaux selon l'invention, également nommés ulcères gastro-intestinaux, sont de préférence des ulcères gastriques ou duodénaux, également nommés ulcères gastro-duodénaux, et plus préférablement des ulcères gastriques. Plus préférablement encore, les ulcères gastriques ou intestinaux selon l'invention sont induits par un principe actif ayant une activité gastrotoxique.

Comme on l'entend ici, l'expression « induits par un principe actif » est synonyme à l'expression « consécutifs à la prise d'un principe actif ».

Comme on l'entend ici, l'expression « principe actif ayant une activité gastrotoxique » regroupe l'ensemble des principes actifs de médicaments susceptibles de provoquer des ulcères gastriques ou intestinaux selon l'invention, notamment comme effet secondaire. A ce titre, *Helicobacter pylori* n'est bien sûr pas un principe actif ayant une activité gastrotoxique au sens de l'invention.

L'homme du métier sait parfaitement identifier les médicaments, et leurs principes actifs associés, qui présentent des effets secondaires gastrotoxiques. En particulier un principe actif ayant une activité gastrotoxique selon l'invention présente une activité d'inhibition de cyclooxygénase, notamment de cyclooxygénase de type 1 (COX-1) et/ou de type 2 (COX-2).

Ainsi, le principe actif ayant une activité gastrotoxique selon l'invention est de préférence un anti-inflammatoire non-stéroïdien (AINS) ou l'aspirine. A titre d'exemple d'AINS selon l'invention on peut notamment citer l'ibuprofène. Au sens de l'invention, le terme « aspirine » regroupe l'acide acétylsalicylique et l'ensemble de ses sels pharmaceutiquement acceptables, tels que ses sels de lysine, et notamment l'acétylsalicylate de DL-lysine.

De préférence, le principe actif ayant une activité gastrotoxique selon l'invention est administré sous forme d'une gélule, d'un comprimé ou d'un sachet.

Comme on l'entend ici, l'expression « cellules de levure » regroupe des cellules de levure viables ou mortes, entières ou sous forme de débris. De préférence, au moins une partie des cellules de levure selon l'invention sont viables.

La viabilité d'une cellule de levure est définie comme la capacité d'une cellule de levure à se multiplier. Le nombre de cellules viables dans un échantillon peut être estimé en déterminant le nombre d'Unités Formant Colonie (UFC) comprises dans l'échantillon.

A titre d'exemple, le nombre d'UFC de cellules de levure d'un échantillon liquide comprenant des levures peut être déterminé en étalant un volume déterminé de l'échantillon sur un milieu solide, par exemple gélosé, permettant la croissance des levures et en incubant le milieu solide pendant une durée, par exemple 48 h, et à une température, par exemple 30°C, permettant la croissance de colonies de levures. Le nombre de colonies rapporté au volume étalé sur le milieu solide permet de déterminer le nombre d'UFC compris dans l'échantillon. Un protocole détaillé de la détermination d'UFC conforme à l'invention est notamment décrit dans Toothaker & Elmer (1984) Antimicrobial Agents and Chemotherapy 26:552-556 au paragraphe « *Assay for S. boulardii* ». Par ailleurs, lorsque l'échantillon de levure se présente sous forme d'un solide, par exemple une poudre lyophilisée, on préfère déterminer le nombre d'UFC compris dans l'échantillon après avoir repris une masse déterminée de l'échantillon dans une solution aqueuse, notamment de l'eau distillée ou une solution à 0,9 % de NaCl à pH 7.

Une « levure » selon l'invention est un champignon de préférence unicellulaire. Ainsi, les cellules de levure selon l'invention sont de préférence du genre *Saccharomyces,* plus préférablement de l'espèce *Saccharomyces cerevisiae* et plus préférablement encore de l'espèce *Saccharomyces boulardii*. *Saccharomyces boulardii* est bien connu de l'homme du métier et est notamment décrit dans Hennequin et al. (2001) J. Clin. Microbiol. 39:551-559.

De manière particulièrement préférée, les cellules de *Saccharomyces boulardii* selon l'invention sont obtenues à partir de médicaments de marque Ultra-Levure® ou à partir des dépôts effectués à *l'American Type Culture Collection* (ATCC, USA) sous la référence 74012 ou à la Collection Nationale de Culture et de Microorganismes (CNCM, France) sous la référence l-745.

De préférence, également les cellules de levure selon l'invention sont lyophilisées.

De manière avantageuse, la viabilité et la vitalité de cellules de levure obtenues à partir de lyophilisats sont supérieures à ce qui peut être obtenu à partir d'autre mode de conservation des cellules de levure.

Comme on l'entend ici, la « lyophilisation » est une méthode de conservation dans laquelle les cellules de levure sont congelées puis soumises à une sublimation de l'eau congelée qu'elles contiennent pour donner un lyophilisat sous forme d'une poudre de levure sèche contenant de préférence moins de 2 % d'eau et plus préférablement moins de 1 % d'eau. De préférence, les cellules de levure lyophilisées sont obtenues à partir de concentrés de cellules de levure. N'importe quel type de méthode de lyophilisation de cellules de levure connu de l'homme du métier peut être utilisé. Toutefois, les cellules de levure sont de préférence lyophilisées selon l'invention à l'aide de la méthode de lyophilisation suivante :
- cultiver les cellules de levure dans un milieu nutritif liquide jusqu'à ce que les cellules atteignent une phase stationnaire;
- concentrer les cellules de levure cultivées et congeler le concentré;
- lyophiliser le concentré.

De préférence les cellules de levure selon l'invention sont administrées sous forme de gélules ou de sachets.

De préférence également, les cellules de levure selon l'invention sont administrées à une dose de 0,5.10⁸ à 100.10¹⁰ UFC/kg/j ou à une dose de 0,00125 g/kg/j à 25 g/kg/j.

Comme l'homme du métier le comprendra la quantité de cellules de levure à administrer par unité de masse (kg) se réfère à la masse de l'individu auquel sont destinées les cellules de levure. Par ailleurs, lorsque la quantité de cellules de levure à administrer est exprimée en unité de masse (g), les cellules de levure sont de préférence sous la forme d'un lyophilisat.

De préférence, la composition pharmaceutique telle que définie ci-dessus et la trousse telle que définie ci-dessus, sont utilisées pour la prévention ou le traitement des ulcères gastriques ou intestinaux induits par le principe actif qu'elles contiennent.

Par ailleurs, comme cela apparaîtra clairement à l'homme du métier, lorsque la trousse selon l'invention est utilisée à titre de médicament, les cellules de levure selon l'invention et le principe actif ayant une activité gastrotoxique selon l'invention peuvent être administrés ensemble, ou bien être administrés de manière séparée, c'est-à-dire selon des voies d'administration distinctes et/ou des régimes d'administration distincts.

A titre d'exemple de trousse selon l'invention, on peut citer une boîte de médicament ou une plaquette de médicament comprenant, d'une part, au moins une composition comprenant des cellules de levure selon l'invention, par exemple une gélule ou un sachet, et, d'autre part, au moins une composition distincte comprenant un principe actif ayant une activité gastrotoxique selon l'invention, par exemple une gélule, un comprimé ou un sachet.

### Exemple

Les inventeurs ont déterminé l'effet anti-ulcéreux de *Saccharomyces boulardii* dans le modèle de l'ulcère gastrique induit par l'ibuprofène chez le rat selon le protocole d'Abraham et al. (2005) Dig Dis Sci 50:1632-1640.

### 1. Matériel et méthodes

### 1.1. Animaux

Des rats mâles Wistar de chez Janvier de poids compris entre 200 et 220 grammes sont utilisés après acclimatation d'au moins 7 jours dans l'animalerie (t°= 22 ± 2°C ; hygrométrie : 50 ± 20 % ; nourriture SAFE "A04" ; cycle nycthéméral : 12 heures de lumière et 12 heures d'obscurité).

### 1.2. Protocole

A J1, le rat reçoit le produit étudié ou le solvant par voie orale. Puis, 1 heure après, le rat reçoit l'ibuprofène à 100 mg/kg par voie orale, ou le solvant pour le groupe témoin. Les rats reçoivent ces traitements pendant 6 jours consécutifs.

Les animaux sont à jeun de nourriture 14-16 heures avant la dernière administration d'ibuprofène, mais pas de boisson. Ils sont placés individuellement dans une cage métallique dont le plancher est grillagé pour éviter la coprophagie.

A J6 le rat est sacrifié. L'estomac est alors prélevé, puis ouvert selon la grande courbe, rincé avec du NaCl à 0.9 %, et placé dans 10 ml de formaline 2 % pour fixer les tissus.

L'évaluation macroscopique des lésions gastriques est effectuée comme suit :
- l'estomac est étalé sur un support en liège avec des épingles ;
- on compte le nombre d'ulcère sur chaque estomac ;
- on mesure la surface ulcérée (mm²) des lésions hémorragiques avec une grille de carrés (x10) et un stéréo-microscope (Leica Wild M8) par les mesures de la longueur (mm) et de la largeur (mm) de chaque ulcération.

### 1.3. Produits

*Saccharomyces boulardii* sous forme lyophilisée (Ultra-Levure®, Biocodex, France, lot 3490) et l'ibuprofène (sel de sodium, réf 11892 Sigma) sont solubilisés dans de l'eau distillée.

Le lot de *Saccharomyces boulardii* utilisé présente une viabilité de 4.10¹⁰ UFC/g et une quantité de 1 mg/ml de levure correspond à une concentration de 4.10⁷ UFC/ml. Les valeurs d'unités formant colonies (UFC) rapportées sont déterminées comme indiqué précédemment en suivant le protocole de Toothaker & Elmer (1984) Antimicrobial Agents and Chemotherapy 26:552-556. La détermination de la viabilité a été effectuée à l'aide de la technique de comptage sur plaque.

La formaline à 10 % est diluée 5 fois (flacon de 15 ml, réf HT 50-1-1, Sigma).

### 1.4 Statistiques

Le test statistique utilisé est une analyse de variance. Quand le résultat ne dépend pas du hasard au seuil de 5 %, on effectue un test de comparaison des groupes traités par rapport au groupe témoin pour déterminer les groupes traités qui diffèrent significativement du groupe témoin au seuil de 5 %.

### 2. Résultats

Les traitements par voie orale de *Saccharomyces boulardii* à 0,3-1,0-3,0 g/kg/j et/ou d'ibuprofène à 100 mg/kg/j ne modifient pas l'évolution du poids corporel des rats par rapport à celui du groupe sans traitement.

Dans le groupe témoin et dans ceux recevant uniquement *Saccharomyces boulardii*, on n'observe aucun ulcère sur la muqueuse gastrique (**Figures 1** **et** **2**).

Par contre, l'ibuprofène à 100 mg/kg/j pendant 6 jours a provoqué l'apparition de 6,60 ± 1,06 ulcères (**Figure 1**) sur la muqueuse, pour une surface totale de 9,40 ± 1,72 mm² (**Figure 2**).

La co-administration de *Saccharomyces boulardii* à 0.3-1.0-3.0 g/kg/j avec l'ibuprofène montre une diminution dose-dépendante du nombre d'ulcère et de la surface d'ulcération, avec respectivement, 5,33 ± 0,92 ; 4,32 ± 1,21 ; 2,39 ± 0,64 ulcères (**Figure 1**), et 6,94 ± 1,41 ; 5,10 ± 1,30 ; 2,44 ± 0,65 mm² (**Figure 2**). A la dose la plus forte, *Saccharomyces boulardii* entraîne une diminution significative de 64 % du nombre d'ulcère et de 74 % de la surface ulcérée.

En conclusion, la co-administration de *Saccharomyces boulardii* et d'ibuprofène pendant 6 jours consécutifs montre que la levure possède des propriétés antiulcéreuses dans le modèle de l'ulcère induit par l'ibuprofène chez le rat.

## Revendications

1. Cellules de levure pour leur utilisation dans la prévention ou le traitement des ulcères gastriques ou intestinaux induits par un principe actif de médicament ayant une activité gastrotoxique et susceptible de provoquer des ulcères gastriques ou intestinaux comme effet secondaire.

2. Cellules de levure pour leur utilisation selon la revendication 1, dans la prévention ou le traitement des ulcères gastriques ou duodénaux.

3. Cellules de levure pour leur utilisation selon la revendication 1 ou 2, dans la prévention ou le traitement des ulcères gastriques.

4. Cellules de levure pour leur utilisation selon l'une des revendications 1 à 3, le principe actif présentant une activité d'inhibition de cyclooxygénase de type 1 (COX-1) et/ou de type 2 (COX-2).

5. Cellules de levure pour leur utilisation selon l'une des revendications 1 à 4, les ulcères étant induits par un anti-inflammatoire non-stéroïdien (AINS) ou l'aspirine.

6. Cellules de levure pour leur utilisation selon l'une des revendications 1 à 5, les ulcères étant induits par l'ibuprofène.

7. Cellules de levure pour leur utilisation selon l'une des revendications 1 à 6, les cellules de levure étant du genre *Saccharomyces.*

8. Cellules de levure pour leur utilisation selon l'une des revendications 1 à 7, les cellules de levure étant de l'espèce *Saccharomyces boulardii.*

9. Cellules de levure pour leur utilisation selon l'une des revendications 1 à 8, les cellules de levure étant lyophilisées.

10. Cellules de levure pour leur utilisation selon l'une des revendications 1 à 9, les cellules de levure étant administrées sous forme de gélules ou de sachets.

11. Cellules de levure pour leur utilisation selon l'une des revendications 1 à 10, les cellules de levure étant administrées à une dose de 0,5.10⁸ à 100.10¹⁰ UFC/kg/j.

12. Composition pharmaceutique comprenant à titre de substances actives :
- des cellules de levure de l'espèce *Saccharomyces boulardii,* éventuellement lyophylisées, et
- au moins un principe actif de médicament ayant une activité gastrotoxique et susceptible de provoquer des ulcères gastriques ou intestinaux comme effet secondaire,
éventuellement en association avec un véhicule pharmaceutiquement acceptable.

13. Composition pharmaceutique selon la revendication 12, dans laquelle le principe actif est tel que défini dans l'une des revendications 4 à 6.

14. Trousse comprenant :
- dans un premier compartiment des cellules de levure ;
- dans un deuxième compartiment au moins un principe actif de médicament ayant une activité gastrotoxique et susceptible de provoquer des ulcères gastriques ou intestinaux comme effet secondaire.

15. Trousse selon la revendication 14, dans laquelle les cellules de levure sont telles que définies dans l'une des revendications 7 à 9.

16. Trousse selon la revendication 14 ou 15, dans laquelle le principe actif est tel que défini dans l'une des revendications 4 à 6.

17. Trousse selon l'une des revendications 14 à 16, pour son utilisation à titre de médicament.

## Patentansprüche

1. Hefezellen zur Verwendung in der Vorbeugung gegen oder der Behandlung von Magen- oder Darmgeschwüren, die durch einen Arzneimittelwirkstoff hervorgerufen wurden, der eine gastrotoxische Wirkung hat und als Nebenwirkung Magen- oder Darmgeschwüre hervorrufen kann.

2. Hefezellen zur Verwendung nach Patentanspruch 1 in der Vorbeugung gegen oder der Behandlung von Magen- und Zwölffingerdarmgeschwüren.

3. Hefezellen zur Verwendung nach Patentanspruch 1 oder 2 in der Vorbeugung gegen oder der Behandlung von Magengeschwüren.

4. Hefezellen zur Verwendung nach einem der Patentansprüche 1 bis 3, wobei der Wirkstoff eine Inhibitorwirkung auf Zyklooxygenase vom Typ 1 (COX-1) und/oder Typ 2 (COX-2) zeigt.

5. Hefezellen zur Verwendung nach einem der Patentansprüche 1 bis 4, wobei die Geschwüre durch ein nichtsteroidales Antirheumatikum (NSAR) oder durch Aspirin hervorgerufen wurden.

6. Hefezellen zur Verwendung nach einem der Patentansprüche 1 bis 5, wobei die Geschwüre durch Ibuprofen hervorgerufen wurden.

7. Hefezellen zur Verwendung nach einem der Patentansprüche 1 bis 6, wobei die Hefezellen der Gattung *Saccharomyces* angehören.

8. Hefezellen zur Verwendung nach einem der Patentansprüche 1 bis 7, wobei die Hefezellen der Art *Saccharomyces boulardii* angehören.

9. Hefezellen zur Verwendung nach einem der Patentansprüche 1 bis 8, wobei die Hefezellen lyophilisiert sind.

10. Hefezellen zur Verwendung nach einem der Patentansprüche 1 bis 9, wobei die Hefezellen in Form von Gelatinekapseln oder Tüten verabreicht werden.

11. Hefezellen zur Verwendung nach einem der Patentansprüche 1 bis 10, wobei die Hefezellen in einer Dosierung von 0,5.10⁸ bis 100.10¹⁰ KbE/kg/d verabreicht werden.

12. Pharmazeutische Zubereitung. als Wirkstoffe enthaltend:
- Hefezellen der Art *Saccharomyces boulardii,* gegebenenfalls lyophilisiert, und
- mindestens einen Arzneimittelwirkstoff, der eine gastrotoxische Wirkung hat und als Nebenwirkung Magen- oder Darmgeschwüre hervorrufen kann,
gegebenenfalls in Verbindung mit einem pharmazeutisch annehmbaren Vehikel.

13. Pharmazeutische Zubereitung nach Patentanspruch 12, wobei der Wirkstoff der Definition eines der Patentansprüche 4 bis 6 entspricht.

14. Etui, enthaltend:
- in einem ersten Fach Hefezellen;
- in einem zweiten Fach mindestens einen Arzneimittelwirkstoff, der eine gastrotoxische Wirkung hat und als Nebenwirkung Magen- oder Darmgeschwüre hervorrufen kann.

15. Etui nach Patentanspruch 14, in dem die Hefezellen der Definition eines der Patentansprüche 7 bis 9 entsprechen.

16. Etui nach einem der Patentansprüche 14 oder 15, in dem der Wirkstoff der Definition eines der Patentansprüche 4 bis 6 entspricht.

17. Etui nach einem der Patentansprüche 14 bis 16 zur Verwendung als Arzneimittel.

## Claims

1. Yeast cells for use in the prevention or treatment of gastric or intestinal ulcers induced by a medicament active principle having gastrotoxic activity and which may cause gastric or intestinal ulcers as a side-effect.

2. Yeast cells for use according to claim 1, in the prevention or treatment of gastric or duodenal ulcers.

3. Yeast cells for use according to claim 1 or 2, in the prevention or treatment of gastric ulcers.

4. Yeast cells for use according to any of claims 1 to 3, the active principle displaying an activity of inhibition of cyclooxygenase of type 1 (COX-1) and/or of type 2 (COX-2).

5. Yeast cells for use according to any of claims 1 to 4, the ulcers being induced by a non-steroidal anti-inflammatory drug (NSAID) or aspirin.

6. Yeast cells for use according to any of claims 1 to 5, the ulcers being induced by ibuprofen.

7. Yeast cells for use according to any of claims 1 to 6, the yeast cells being of the genus *Saccharomyces.*

8. Yeast cells for use according to any of claims 1 to 7, the yeast cells being of the species *Saccharomyces boulardii.*

9. Yeast cells for use according to any of claims 1 to 8, the yeast cells being lyophilized.

10. Yeast cells for use according to any of claims 1 to 9, the yeast cells being administered in the form of capsules or sachets.

11. Yeast cells for use according to any of claims 1 to 10, the yeast cells being administered at a dose from 0.5×10⁸ to 100×10¹⁰ CFU/kg/d.

12. A pharmaceutical composition comprising as active substances:
- yeast cells of the *Saccharomces boulardii* species, optionally lyophilized, and
- at least one medicament active principle having gastrotoxic activity and which may cause gastric or intestinal ulcers as a side-effect,
optionally together with a pharmaceutically acceptable vehicle.

13. The pharmaceutical composition according to claim 12, wherein the active principle is as defined in any of claims 4 to 6.

14. A kit comprising:
- in a first compartment, yeast cells;
- in a second compartment, at least one medicament active principle having gastrotoxic activity and which may cause gastric or intestinal ulcers as a side-effect.

15. The kit according to claim 14, wherein the yeast cells are as defined in any of claims 7 to 9.

16. The kit according to claim 14 or 15, wherein the active principle is as defined in any of claims 4 to 6.

17. The kit according to any of claims 14 to 16, for use as a medicament.
